# EUROPEAN PATENT APPLICATION

(11) **EP 2 394 652 A2**
(43) Date of publication of application: **14.12.2011**
(21) Application number: 11005763.5
(22) Date of filing: 03.02.2005
(51) Int. Cl.: A61K 31/541, A61K 39/395, A61K 31/381, A61P 35/00, C07K 16/00, C07K 16/28

(54) **Methods and compositions for treating tumors and metastatic disease**

(30) Priority: 06.02.2004 US 541946
(62) Divisional of application: 05712339.0
(71) Applicant: Elan Pharmaceuticals Inc., South San Francisco, CA 94080 (US)
(72) Inventor: Wehner, Nancy, Fremont, California 94536 (US)
(74) Representative: Schüssler, Andrea

(57) **Abstract**

Compositions, methods, and combination therapies for the treatment of lymphomas, leukemias, melanomas, prostate cancer, and metastatic disease are provided. Specifically, compositions comprising anti-α4 integrin immunoglobulins or immunoglobulins that bind to an α4 integrin ligand (e.g., MadCAM-1 and VCAM-1) are disclosed for use in inhibiting tumor growth and progression and inhibition of metastases. A preferred immunoglobulin for use in treating tumors and metastases is natalizumab. The compositions and methods using these immunoglobulins can be used alone or in combination with other reagents and cancer treatment modalities.

## Description

### CROSS REFERENCE TO RELATED APPLICATIONS

This application claims the benefit of U.S. Provisional Application No. 60/541,946, filed February 6, 2004.

### FIELD OF THE INVENTION

The application relates to a method of using an anti-α4 integrin immunoglobulin or an immunoglobulin to an α4 integrin ligand (*e.g*., MadCAM-1 and VCAM-1) to treat subjects suffering from a malignancy and/or metastatic disease that involves expression of an α4 integrin. The use of the preparation of a medicament to inhibit tumor growth and/or metastatic progression and/or development of metastases is also provided.

### BACKGROUND OF THE INVENTION

Both benign and malignant tumors are known to express proteins in patterns not found in normal cells. The pattern of proteins exhibited by tumor or malignant cells can reflect the stage of disease (*i.e*., early stage or metastatic disease). As a malignancy progresses, the cells tend to differ more and more from the tissue that they originated from. As a cancer progresses becoming more undifferentiated, regardless of the staging schema used to determine the cancer's progression, the cells become more likely to metastasize and/or are more refractory to treatment by traditional therapies. Cancer therapies may include one or more of the following treatments: chemotherapy, surgery, radiation treatment, hyperthermia, immunotherapy, bone marrow transplant, hormone therapy, and biotherapy.

Integrins are a family of cell-surface glycoproteins involved in cell-adhesion, immune cell migration and activation. Alpha-4 integrin is expressed by all circulating leukocytes except neutrophils, and forms heterodimeric receptors in conjunction with either the beta-1 (β1) or beta-7 (β7) integrin subunits. Both alpha-4 beta-1 (α4β1) integrin and alpha-4 beta-7 (α4β7) integrin play a role in migration of leukocytes across the vascular endothelium (Springer et al., Cell, 1994, 76: 301-14; Butcher et al., Science, 1996, 272: 60-6) and contribute to cell activation and survival within the parenchyma (Damle et al., J. Immunol., 1993; 151: 2368-79; Koopman et al., J. Immunol., 1994, 152: 3760-7; Leussink et al., Acta Neuropathol., 2002, 103: 131-136). α4β1 integrin is constitutively expressed on lymphocytes, monocytes, macrophages, mast cells, basophils, and eosinophils.

Alpha-4 beta-1 (also known as very late antigen-4, VLA-4), binds to vascular cell adhesion molecule-1 (VCAM-1) (Lobb et al., J. Clin. Invest. 1994, 94: 1722-8), which is expressed by the vascular endothelium at many sites of chronic inflammation (Bevilacqua et al., 1993, Annu. Rev. Immunol., 11: 767-804; Postigo et al., 1993, Res. Immunol., 144: 723-35). α4β1 integrin has other ligands, including fibronectin and other extracellular matrix (ECM) components.

Alpha-4 beta-7 integrin interacts with mucosal addressin cell adhesion molecule (MAdCAM-1), and mediates homing of lymphocytes to the gut (Farstad et al., 1997, Am. J. Pathol., 150: 187-99; Issekutz, 1991, J. Immunol. 147: 4178-84). Thus, new agents, compositions and methods for using these agents and compositions that inhibit cancer growth and metastasis are needed, which can be used alone or in concert with other agents to treat cancer, especially advanced stage tumors, which typically involve metastases.

### SUMMARY OF THE INVENTION

The invention provides for new methods, compositions, and combination therapies for treating tumors and/or metastatic disease and/or inhibiting growth of tumors. The methods, compositions and combination therapies are preferably directed towards the treatment of α4 expressing cancers such as lymphomas, leukemias, melanomas, prostate cancer, and metastatic disease of any α4 expressing primary tumor.

Accordingly, one aspect of the invention provides for a method for inhibiting tumor growth and/or metastases or metastatic spread comprising administering an anti-α4 immunoglobulin or an immunoglobulin to an α4 integrin ligand (*e.g*., MadCAM-1 and VCAM-1) to a subject in need thereof in an amount sufficient to inhibit tumor growth, and/or metastases, and/or metastatic spread. The anti-α4 antibody preferably binds to α4β1 integrin and/or α4β7 integrin, and more preferably the immunoglobulin is a monoclonal antibody (*e.g*., natalizumab).

It is yet a further aspect of the invention that the tumor treated is a solid tumor or a soft tissue tumor. Solid tissue tumors contemplated for treatment using the methods, combination therapies, and anti-α4 integrin immunoglobulins or immunoglobulins against α4 integrin ligands include but are not limited to melanomas (*e.g*., cutaneous melanoma, a metastatic melanoma, or an intraocular melanoma), prostate cancers, and metastatic lesions of other primary tumors.

Another aspect of the invention contemplates that the tumor to be treated is a soft tissue tumor such as a bone tumor, a leukemia (*e.g*., chronic myelogenous leukemia, acute myelogenous leukemia, adult acute lymphoblastic leukemia, acute myelogenous leukemia, mature B-cell acute lymphoblastic leukemia, chronic lymphocytic leukemia, prolymphocytic leukemia, or hairy cell leukemia), or a lymphoma (*e.g*., a non-Hodgkin's lymphoma, a cutaneous T-cell lymphoma, or Hodgkin's disease).

A further embodiment of the invention contemplates that the anti-α4 integrin immunoglobulin is administered to the subject in an amount of about 1 mg/kg subject weight to about 100 mg/kg subject weight (and any integer value falling within this range). More preferably, the anti-α4 integrin immunoglobulin is administered to the subject in an amount of about 1 mg/kg subject weight to about 10 mglkg subject weight. Preferably, the immunoglobulin is natalizumab.

In a further aspect of the invention, the immunoglobulins can be administered alone or in combination with other cancer modalities in a multimodality format. For example, if the tumor is a melanoma, the subject can be administered natalizumab after having had the melanoma surgically removed. If the tumor is melanoma, the above method can be further combined with such cancer modalities as isolated limb perfusion, regional chemotherapy infusion, systemic chemotherapy, or immunotherapy with a second antibody (*e.g*., an anti-GM2 ganglioside antibody, anti-GD2 ganglioside antibody, anti-GD3 ganglioside antibody), or antisera. The chemotherapeutic agent can be any one or more of the following: dacarbazine, carmustine, lomustine, tauromustine, fotemustine, semustine, cisplatin, carboplatin, vincristine, vinblastine, vindesine, taxol, dibromodulcitol, detorubicin, piritrexim and interferon (*e.g*., interferon-a2).

Another aspect of the invention contemplates a method of treating metastases to the brain, lung, liver, or bone.

Yet another aspect of the invention contemplates a method of treating lymphomas using immunoglobulins to α4 integrins or their ligands (*e.g*., MadCAM-1 and VCAM-1) in combination with other lymphomas treatment modalities.

Another aspect of the invention contemplates a combination therapy wherein immunoglobulins to α4 integrin or its ligands (*e.g*., MadCAM-1 and VCAM-1) are used in combination with other tumor treatment modalities as known in the art. It is yet a further aspect of the invention to provide for a use for the preparation of a medicament to inhibit tumor growth, and/or inhibit metastasis, and/or inhibit or slow disease progression when administered to a subject in need thereof comprising an anti-α4 integrin immunoglobulin or an immunoglobulin to a α4 integrin ligand (*e.g*., MadCAM-1 and VCAM-1).

A further aspect of the invention contemplates a use for the preparation of a medicament to inhibit tumor growth and/or metastatic progression and/or development of metastases when administered to a subject in need thereof, comprising an anti-α4 integrin immunoglobulin. Preferably, the subject is further treated with a chemotherapy, an immunotherapy, surgery, radiation therapy, hyperthermia, or a drug to ameliorate the adverse effects of a cancer therapy. Preferably, the tumor is a melanoma, a leukemia, a prostate cancer, or a lymphoma.

### BRIEF DESCRIPTION OF DRAWINGS

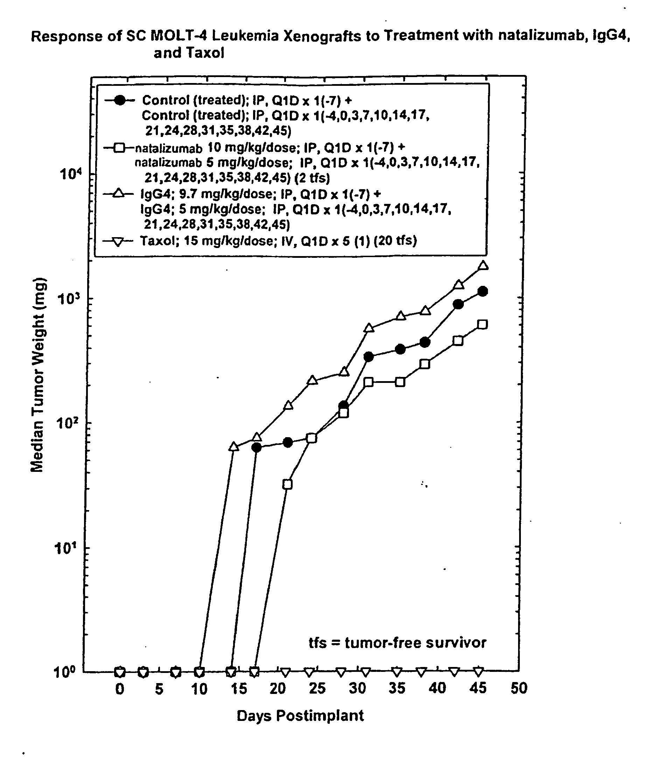

Figure 1 is a graph showing the primary tumor growth of MOLT-4 xenografts upon administration of a control, taxol, IgG4 or natalizumab after establishment of the tumor. SCID mice were treated with Saline (N=20), natalizumab (N=20), IgG4 (N=20), or Taxol (N=20). Saline, natalizumab and IgG4 were administered on Days -7, -4, 0, 3, 7, 10, 14, 17, 21, 24, 28, 31, 35, 38, 42, and 45. MOLT-4 leukemia xenografts were implanted subcutaneously on day 0. Taxol was administered intravenously on Days 1-5. Tumors were measured twice weekly.

### DETAILED DESCRIPTION OF THE INVENTION

### 1: Definitions and Acronyms

In accordance with this detailed description, the following abbreviations and definitions apply. It must be noted that as used herein, the singular forms "a", "and", and "the" include plural referents unless the context clearly dictates otherwise. Thus, for example, reference to "an antibody" includes a plurality of such antibodies and reference to "the dosage" includes reference to one or more dosages and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates, which may need to be independently confirmed.

### 1.1 Definitions

By the term "subject" or "patient" as used herein is meant to include a mammal. The mammal can be a canine, feline, primate, bovine, ovine, porcine, camelid, caprine, rodent, or equine. Preferably the mammal is human.

By "natalizumab" or "Tysabri®" is meant a humanized antibody against VLA-4 as described in U.S. Patent Nos. 5,840,299 and 6,033,665, which are herein incorporated by reference in their entirety. Also contemplated herein are other VLA-4 specific antibodies. Such anti-VLA-4 antibodies and immunoglobulins include but are not limited to those immunoglobulins described in U.S. Patent Nos. 6,602,503 and 6,551,593, published U.S. Application No. 20020197233 (Relton et al.). Preparation of the antibody can be by the methods disclosed in these patents and applications, by mammalian cell expression, or via transgenic animal expression systems (*e.g*., goat).

The term "efficacy" as used herein refers to the effectiveness of a particular treatment regime. Efficacy can be measured based on such characteristics (but not limited to these) as inhibition of tumor growth, reduction of tumor mass, reduction of metastatic lesions as assessed, for example, by radiologic imaging, slowed tumor growth, lack of detectable tumor associated antigens, and the like. Additional methods of assessing tumor progression are discussed herein and would be known to the treating and diagnosing physicians.

By the phrases "pharmaceutically acceptable carrier" and "pharmaceutically acceptable excipient" are intended to mean any compound(s) used in forming a part of the formulation that is intended to act merely as a carrier, *i.e*., not intended to have biological activity itself. The pharmaceutically acceptable carrier or excipient is generally safe, non-toxic, and neither biologically nor otherwise undesirable. A pharmaceutically acceptable carrier or excipient as used herein includes both one and more than one such carrier or excipient.

The terms "treating", and "treatment", and the like are used herein to generally mean obtaining a desired pharmacological and physiological effect. More specifically, the reagents described herein which are used to treat a subject with a tumor and metastatic disease generally are provided in a therapeutically effective amount to achieve any one or more of the following: inhibited tumor growth, reduction in tumor mass, loss of metastatic lesions, inhibited development of new metastatic lesions after treatment has started, or reduction in tumor such that there is no detectable disease (as assessed by *e.g*., radiologic imaging, biological fluid analysis, cytogenetics, fluorescence in situ hybridization, immunocytochemistry, colony assays, multiparameter flow cytometry, or polymerase chain reaction). The term "treatment", as used herein, covers any treatment of a disease in a mammal, particularly a human.

By "therapeutically effective amount" is meant an amount of an agent, reagent, compound, composition, or combination of reagents disclosed herein that when administered to a mammal is sufficient to be effective against the tumor.

By the term "tumor" is meant to include both benign and malignant growths or cancer. Thus, the term "cancer", unless otherwise stated, can include both benign and malignant growths. Preferably, the tumor is malignant The tumor can be a solid tissue tumor such as a melanoma, or a soft tissue tumor such as a lymphoma, a leukemia, or a bone cancer.

By the term "primary tumor" is meant the original neoplasm and not a metastatic lesion located in another tissue or organ in the patient's body. By the terms "metastatic disease", "metastases", and "metastatic lesion" are meant a group of cells which have migrated to a site distant relative to the primary tumor.

### 1.2 Acronyms

The following acronyms are commonly used for the associated terms and would be known in the art.
- α4β1: alpha-4 beta-1
- α4β1: alpha-4 beta-7
- Ab: antibody
- ABDIC: doxorubicin, bleomycin, dacarbazine, lomustine, and prednisone
- ALL: acute lymphocytic leukemia
- AML: acute myelogenous leukemia
- BMT: bone marrow transplant
- CAF: cyclophosphamide, adriamycin, and 5-fluorouracil
- CAMP: lomustine, mitoxantrone, cytarabine, and prednisone
- CAVP: lomustine, melphalan, etoposide, and prednisone
- CBVD: lomustine, bleomycin, vinblastine, dexamethasone
- CCNU: lomustine
- CEFF(B): cyclophosphamide, etoposide, procarbazine, prednisone, and bleomycin
- CEM: lomustine, etoposide, and methotrexate
- CEP: lomustine, etoposide, and prednimustine
- CEVD: lomustine, etoposide, vindesine, and dexamethasone
- CHOP: cyclophosphamide, doxorubicin, vincristine, and prednisone
- CLL: chronic lymphocytic leukemia
- CMF: cyclophosphamide, methotrexate, and 5-fluorouracil
- CML: chronic myelogenous leukemia
- CNS: central nervous system
- CTCL: cutaneous T-cell lymphoma
- DHAP: dexamethasone, high dose cytarabine, and cisplatin
- ECM: extracellular matrix
- EPOCH: etoposide, vincristine, doxorubicin, cyclophosphamide, and prednisone
- ESHAP: etoposide, methylpredisolone, high dose (HD) cytarabine, and cisplatin
- EVA: etoposide, vinblastine, and doxorubicin
- EVAP: etoposide, vinblastine, cytarabine, and cisplatin
- GAP-BOP: cyclophosphamide, doxorubicin, procarbazine, bleomycin, vincristine, and prednisone
- HD: high dose
- 3H-FUDR: 3H-floxuridine
- IFN: interferon
- IFN-a2: interferon-a2
- IFNβ-1a: interferon β-1a
- IHC: immunohistochemistry
- i.m.: intramuscular
- IMVP-16: ifosfamide, methotrexate, and etoposide
- i.p.: intraperitoneal
- i.v.: intravascular
- m-BACOD: methotrexate, bleomycin, doxorubicin, cyclophosphamide, vincristine, dexamethasone, and leucovorin
- MAb: monoclonal antibody
- MACOP-B: methotrexate, doxorubicin, cyclophosphamide, vincristine, prednisone, bleomycin, and leucovorin
- MadCAM-1: mucosal addressin cell adhesion molecule 1 (also known as CD106)
- MeCCNU: semustine or methyl CCNU
- MF: mycosis fungoides
- MIME: methyl-gag, ifosfamide, methotrexate, and etoposide
- MINE: mitoquazone, ifosfamide, vinorelbine, and etopside
- MOPLACE: cyclophosphamide, etoposide, prednisone, methotrexate, cytarabine, and vincristine
- MOPP: mechlorethamine, vincristine, procarbazine, and prednisone
- MS: multiple sclerosis
- MTX: methotrexate
- MTX-CHOP: methotrexate and CHOP
- NAT: natalizumab (Tysabri®)
- PBMC: peripheral blood monocytic cells
- PCVP: vinblastine, procarbazine, cyclophosphamide, and prednisone
- p.o.: oral administration (per os)
- ProMACE-MOPP: prednisone, methotrexate, doxorubicin, cyclophosphamide, etoposide, leucovorin with standard MOPP
- s.c.: subcutaneous
- SDS-PAGE: sodium dodecyl sulfate polyacrylamide gel electrophoresis
- SCID: severe combined immunodeficiency
- TNM: tumor, node, and metastases is the American Joint Commission on Cancer staging classification
- VABCD: vinblastine, doxorubicin, dacarbazine, lomustine, and bleomycin
- VCAM-1: vascular cell adhesion molecule 1 (also known as CD106 and INCAM-110)
- VLA-4: very late antigen 4 (also known as alpha-4 beta-1, α4β1 integrin, VLA-4a, and CD49d)

### 2. Diseases

In one aspect of the invention, the methods and compositions disclosed herein can be used to inhibit or slow the progression of malignancies. These malignancies can be solid or soft tissue tumors. Soft tissue tumors include bone cancers, lymphomas, and leukemias. Another aspect of the invention is to use the methods and compositions to inhibit or prevent metastases or metastatic progression.

Thus, an aspect of the invention is to treat tumors or metastatic disease with an immunoglobulin. This immunoglobulin can target α4 and preferably α4β1 integrin and/or α4β7 integrin. Alternatively, the immunoglobulin can target ligands of α4 (*e.g*., VCAM-1 MadCAM-1). These immunoglobulins can be used alone, in combination with each other, or in combination with other cancer modalities, such as but not limited to chemotherapy, surgery, radiotherapy, hyperthermia, immunotherapy, hormone therapy, biologic therapy (*e.g*., immune effector mechanisms resulting in cell destruction, cytokines, immunotherapy, interferons, interleukin-2, cancer vaccine therapy, and adoptive therapy), and drugs to ameliorate the adverse side effects of such cancer modalities.

### 2.1 Cancer Treatment

The term cancer embraces a collection of malignancies with each cancer of each organ consisting of numerous subsets. Typically, at the time of cancer diagnosis, "the cancer" consists in fact of multiple subpopulations of cells with diverse genetic, biochemical, immunologic, and biologic characteristics.

The types of cancers to be treated by the compositions and methods of the instant invention are those that exhibit α4 integrins (*i.e*., α4β1 and/or α4β7) or their ligands (*e.g*., VCAM-1 and/or MadCAM-1). Preferred cancers include but are not limited to melanomas (*e.g*., cutaneous melanoma, metastatic melanomas, and intraocular melanomas), prostate cancer, lymphomas (*e.g*., cutaneous T-cell lymphoma, mycosis fungicides, Hodgkin's and non-Hodgkin's lymphomas, and primary central nervous system lymphomas), leukemias (*e.g*., pre-B cell acute lymphoblastic leukemia, chronic and acute lymphocytic leukemia, chronic and acute myelogenous leukemia, adult acute lymphoblastic leukemia, mature B-cell acute lymphoblastic leukemia, prolymphocytic leukemia, hairy cell leukemia, and T-cell chronic lymphocytic leukemia), and metastatic tumors which exhibit these proteins on the cell surface. It should be noted that although mycosis fungoides (MF), Sézary syndrome, reticulum cell sarcoma of the skin and several other cutaneous lymphocytic dyscrasias were once considered separate conditions, they are now recognized as different clinical presentations of cutaneous T-cell lymphoma (CTCL) and thus included in the term. *See* Lynn D. Wilson et al., "Cutaneous T-Cell Lymphomas," in CANCER: PRINCIPLES & PRACTICE OF ONCOLOGY 2220-2232 (Vincent T. DeVita, Jr. et al., editors, 5th ed. 1997); Bank et al., 1999, J. Cutan. Pathol., 26(2): 65-71.

### 2.2 Metastatic Disease

Once a tumor is diagnosed in a patient, the first question is whether the tumor has progressed and spread to the regional lymph nodes and to distant organs. In the end, most cancer deaths result from metastases that are resistant to conventional cancer therapies. Metastases can be located in different organs and in different regions of the same organ, making complete eradication by surgery, radiation, drugs, and/or biotherapy nearly impossible.

Also contemplated for treatment with the methods, combination therapies, and compositions disclosed herein is the treatment of metastatic cancer. Cancers typically begin their growth in only one location in the tissue of origin, As the cancer progresses, the cancer may migrate to a distal location in the patient. For example, a cancer beginning in the prostate may migrate to the lung. Other locations common for metastatic disease and that are contemplated herein include metastatic cancer to the brain, lung, liver, and bone. Several integrin subunits (*i.e*., α2, α4 and β3) have been found to have increased expression in metastasis as compared to normal prostate tissue and normal melanocytes. Hartstein et al., 1997, Ophthal. Plast. Reconstr. Surg., 13(4): 227-38.

There are essential steps in the formation of metastasis in all tumors. The steps include the following:
(1) After neoplastic transformation, progressive proliferation of neoplastic cells supported by the organ/tissue environment in which the neoplasm is located.
(2) Neovascularization or angiogenesis of the tumor for further growth beyond 1 to 2 mm in diameter.
(3) Down-regulation of expression of cohesive molecules wherein the cells have increased motility or ability to detach from the primary lesion.
(4) Detachment and embolization of single tumor cells or cell aggregates, with the vast majority of these cells being rapidly destroyed.
(5) Once tumor cells survive the detachment and embolization step, they must go on to proliferate within the lumen of the blood vessel. The cells will then go on to extravasate into the organ parenchyma by mechanism similar to those operative during invasion.
(6) Tumor cells with the appropriate cell surface receptors can respond to paracrine growth factors and hence proliferate in the organ parenchyma.
(7) Tumor cell evasion of host defenses (both specific and nonspecific immune responses).
(8) For a metastasis to proliferate beyond 1 to 2 mm in diameter, the metastases must develop a vascular network.

Thus, if a primary tumor is given enough time to go through these steps, it will form metastatic lesions at a site or sites distant to the primary tumor. The reagents, methods, and combination therapies disclosed inhibit or prevent one or more of these steps in the metastatic process. For additional details on the mechanism and pathology of tumor metastasis, *see* Isaiah J. Fidler, "Molecular Biology of Cancer: Invasion and Metastasis," in CANCER: PRINCIPLES & PRACTICE OF ONCOLOGY 135-152 (Vincent T. DeVita et al., editors, 5th ed., 1997).

Accordingly, one aspect of the invention provides for methods using and compositions comprising anti-α4 integrin immunoglobulins or immunoglobulins that target ligands of α4 integrins (*e.g*., VCAM-1 and MadCAM-1). A preferred anti-α4 integrin immunoglobulin is an anti-VLA-4 antibody such as natalizumab. These immunoglobulins can be used alone or in combination with other agents or cancer treatment modalities that prevent metastases or inhibit progression of metastatic lesions. Thus, the compositions and methods can be used to treat any metastases of any primary tumor that exhibits an α4 integrin or ligands of α4 integrins.

### 3. Immunoglobulin Therapy

The immunoglobulins contemplated for use in treating the above cancers include anti-α4 integrin antibodies which inhibit α4β1 and/or α4β7 from binding to their cognate ligands, *e.g*., VCAM-1 or MAdCAM-1. Also contemplated for use are immunoglobulins against the ligands. These immunoglobulins can be antibodies (*i.e*., monoclonal, primatized, humanized, or human antibodies as well as chimeric antibodies, and bispecific antibodies). The immunoglobulins can also be immunogenic fragments of antibodies (*e.g*., Fab, scFv, Fab', F(ab')2, Fab", Fabc, or a recombinantly synthesized fragment) or recombinantly produced immunoglobulins. A preferred anti-α4 antibody is natalizumab. However, other anti-α4 integrin immunoglobulins are also contemplated, including immunoglobulins which can distinguish between α4β1 and α4β7. Preferred immunoglobulins are monoclonal antibodies, and more preferred immunoglobulins are humanized or primatized antibodies, if the subject patient is human.

These antibodies can be used alone or in combination with other anti-α4 integrin immunoglobulins or immunoglobulins to α4 integrin ligands. Another aspect of the invention contemplates the use of anti-α4 antibodies in combination with other conventional cancer treatment modalities, in the form of combination therapies.

### 4. Combination Therapy

Many treatments exist for cancers. The particular cancer therapy or combination of therapy modalities used to treat a cancer depend greatly on the type of cancer, its stage, the patient (*e.g*., weight, sex, age, health, prior cancers, and the like), and where the patient is in therapy (*e.g*., first treatment, in blast crisis, refractive to initial treatments, cancer relapse, or a second cancer perhaps induced by the treatment of the first cancer months or years before). Therefore, physicians will frequently have to combine a variety of treatment modalities that will best suit the needs of the patient in combating the disease and the patient's self-determination of quality of life. Treatment modalities include but are not limited to surgery, radiation therapy, chemotherapy, biologic therapy (*e.g*., cytokines, immunotherapy, and interferons), hormone therapies, and hyperthermia.

Conventional chemotherapy can be further broken down into hormone therapies (*e.g*., antiestrogens, aromatase inhibitors, gonadotropin-releasing hormone analogues, and anti-androgens), anti-tumor alkylating agents (*e.g*., mustards, nitrosoureas, tetrazines, and aziridines), cisplatin and its analogues, anti-metabolites (*e.g*., methotrexate, antifolates, 5-fluoropyrimidines, cytarabine, azacitidine, gemcitabine, 6-thipurines, and hydroxyurea), topoisomerase interactive agents, antimicrotubule agents (*e.g*., vinca alkaloids, taxanes, and estramustine), differentiating agents (*e.g*., retinoids, vitamin D3, polar-apolar compounds, butyrate and phenylactetate, cytotoxic drugs, cytokines, and combinations thereof), and other chemotherapeutic agents such as fludarabine, 2-chlorodeoxyadenosine, 2'-deoxycoformycin, homoharringtonine (HHT), suramin, bleomycin, and L-asparaginase.

### 4.1 Combination Therapy For Treating Lymphomas

One aspect of the invention contemplates the use of anti-α4 integrin immunoglobulins or immunoglobulins that bind to α4 integrin ligands (*e.g*., MadCAM-1 and VCAM-1) to treat lymphomas. Any lymphoma cell which expresses an α4 integrin or a ligand to an α4 integrin is contemplated for treatment with the combination therapies disclosed herein.

Lymphomas contemplated for treatment by these combination therapies include T-cell lymphomas such as but not limited to cutaneous T-cell lymphoma (CTCL), T-cell non-Hodgkin's lymphoma, peripheral T-cell lymphomas, anaplastic large-cell lymphoma, anti-immunoblastic lymphoma, and precursor T-LBL. Treatment of lymphomas is again dependent on the subject being treated, the type of disease, and its stage. Existing treatment modalities for leukemias and lymphomas are described generally in CANCER: PRINCIPLES & PRACTICE OF ONCOLOGY (Vincent T. DeVita et al., editors, 5th ed., 1997). Also contemplated for treatment are B-cell lymphomas (e.g., follicular lymphoma, diffuse large B-cell lymphoma, mantle cell lymphoma, B-CLL/SLL, immunocytoma/Waldenstrom's, and MALT-type/monocytoid B cell lymphoma). Also contemplated are the treatment of pediatric lymphomas such as Burkitt's lymphoma, diffuse large B-cell lymphoma, follicular lymphoma, precursor B-LBL, precursor T-LBL, anaplastic large cell lymphoma, and peripheral T-cell lymphoma.

Common drug combinations for use in treating lymphomas include but are not limited to CHOP (*i.e*., cyclophosphamide, doxorubicin, vincristine, and prednisone), GAP-BOP (*i.e*., cyclophosphamide, doxorubicin, procarbazine, bleomycin, vincristine, and prednisone), m-BACOD (*i.e*., methotrexate, bleomycin, doxorubicin, cyclophosphamide, vincristine, dexamethasone, and leucovorin), ProMACE-MOPP (*i.e*., prednisone, methotrexate, doxorubicin, cyclophosphamide, etoposide, leucovorin with standard MOPP), ProMACE-CytaBOM (prednisone, doxorubicin, cyclophosphamide, etoposide, cytarabine, bleomycin, vincristine, methotrexate, and leucovorin), and MACOP-B (methotrexate, doxorubicin, cyclophosphamide, vincristine, prednisone, bleomycin, and leucovorin). For relapsed aggressive non-Hodgkin's lymphoma the following chemotherapy drug combinations may be used with the antibodies and drug combinations described herein: IMVP-16 (*i.e*., ifosfamide, methotrexate, and etoposide), MIME (*i.e*., methyl-gag, ifosfamide, methotrexate, and etoposide), DHAP (*i.e*., dexamethasone, high dose cytarabine, and cisplatin), ESHAP (*i.e*., etoposide, methylprednisone, high dosage cytarabine, and cisplatin), CEFF(B) (*i.e*., cyclophosphamide, etoposide, procarbazine, prednisone, and bleomycin), and CAMP (*i.e*., lomustine, mitoxantrone, cytarabine, and prednisone). *See* Margaret A. Shipp, et al., "Non-Hodgkin's Lymphomas," in CANCER: PRINCIPLES & PRACTICE OF ONCOLOGY 2165-2220 (Vincent T. DeVita et al., editors, 5th ed., 1997).

Treatment for salvage chemotherapy used for certain lymphomas such as for relapsed, resistant Hodgkin's Disease include but are not limited to VABCD *(i.e.,* vinblastine, doxorubicin, dacarbazine, lomustine and bleomycin), ABDIC *(i.e.,* doxorubicin, bleomycin, dacarbazine, lomustine, and prednisone), CBVD (*i.e*., lomustine, bleomycin, vinblastine, dexamethasone), PCVP (*i.e*., vinblastine, procarbazine, cyclophosphamide, and prednisone), CEP (*i.e*., lomustine, etoposide, and prednimustine), EVA (*i.e*., etoposide, vinblastine, and doxorubicin), MOPLACE (*i.e*., cyclophosphamide, etoposide, prednisone, methotrexate, cytaravine, and vincristine), MIME (*i.e*., methyl-gag, ifosfamide, methotrexate, and etoposide), MINE (*i.e*., mitoquazone, ifosfamide, vinorelbine, and etoposide), MTX-CHOP (*i.e*., methotrexate and CHOP), CEM (*i.e*., lomustine, etoposide, and methotrexate), CEVD (*i.e*., lomustine, etoposide, vindesine, and dexamethasone), CAVP (*i.e*., lomustine, melphalan, etoposide, and prednisone), EVAP (*i.e*., etoposide, vinblastine, cytarabine, and cisplatin), and EPOCH (*i.e*., etoposide, vincristine, doxorubicin, cyclophosphamide, and prednisone). *See*, *e.g*., Vincent T. DeVita et al., "Hodgkin's Disease," in CANCER: PRINCIPLES & PRACTICE OF ONCOLOGY 2242- 2283 (Vincent T. DeVita et al., editors, 5th ed., 1997).

For CTCL, conventional therapies are dependent on the patient's evaluation. Thus, based on TNM staging, a patient may be treated with topical chemotherapy, psoralen ultraviolet therapy, localized external beam radiotherapy, extracorporeal photochemotherapy, interferon (IFN), systemic chemotherapy with nucleotide derivatives, or photon irradiation in combination with the methods and compositions disclosed herein. Additional treatment modalities are known in the art. *See* generally CANCER: PRINCIPLES & PRACTICE OF ONCOLOGY (Vincent T. DeVita et al., editors, 5th ed., 1997).

Thus, one aspect of the invention contemplates the use of anti-α4 immunoglobulins or immunoglobulins against ligands of-α4, α4β1, and α4β7 to be used to inhibit lymphoma progression in a subject and/or metastasis of a lymphoma. The reagents can be used either alone, or in combination with other lymphoma treatments as discussed herein.

### 4.2 Combination Treatment of Melanomas

Another aspect of the invention contemplates inhibiting melanoma growth and/or inhibiting growth or spread of melanoma metastases. The anti-α4 integrin (*i.e*., α4β1 and α4β7) immunogloublins or immunoglobulins which recognize a ligand of α4 integrin can be used alone or in combination with other cancer modalities for treating melanoma. The methods and compositions are contemplated for but not limited to treating cutaneous melanomas, metastatic melanomas and intraocular-melanomas. Conventional therapies for treating these melanomas are known in the art. *See e.g*., Anthony P. Albino et al., "Molecular Biology of Cutaneous Malignant Melanoma," in CANCER: PRINCIPLES & PRACTICE OF ONCOLOGY 1935-1947 (Vincent T. DeVita et al., editors, 5th ed., 1997); Charles M. Balch et al., "Cutaneous Melanoma," in CANCER: PRINCIPLES & PRACTICE OF ONCOLOGY, 1947-1994; and Jose A. Sahel et al., "Intraocular Melanoma," IN CANCER: PRINCIPLES & PRACTICE OF ONCOLOGY, 1995-2012.

For example, for treatment of metastatic melanoma, use of surgery, isolated limb perfusion, regional chemotherapy infusion (with *e.g*., decarbazine or cisplatin), radiation therapy, immunotherapy (*e.g*., treatment with antibodies against GD2 and GD3 gangliosides), intralesional immunotherapy, systemic chemotherapy, hyperthermia, systemic immunotherapy, tumor vaccines, or combinations thereof can be further combined with the anti-α4 immunogloublins or immunoglobulins against ligands of α4, α4β1, and α4β7.

### 4.3 Combination Therapy For Treating Leukemia

Another aspect of the invention provides for the treatment of certain leukemias using the anti-α4 immunoglobulins or immunoglobulins against α4 ligands (*e.g*., VCAM-1 and MadCAM-1) in combination with conventional treatment modalities for the leukemia to be treated.

Traditional treatment for acute myelogenous leukemia (AML) includes but is not limited to anthracycline/ytarabine-based induction regimens, intensive post-remission therapy such as bone marrow transplant (BMT) or high-dose (HD) cytarabine.

Traditional treatment for acute promyelocytic leukemia includes but is not limited to retinoic acid and anthracycline/cytarabine-based treatment. Patients may also be administered a cryoprecipitate or fresh frozen plasma to maintain fibrinogen levels of greater than 100 mg/dL. Platelet transfusions may also be necessary to maintain a daily platelet count in a human of > 50,000/µL.

Traditional treatment modalities for acute lymphoblastic leukemia (ALL) includes four or five drug induction regimens using anthracyclines, cyclophosphamide, asparaginase or a combination in addition to vincristine and prednisone. Alternatively, the physician may opt to use an intensive consolidation therapy based on cytarabine combined with anthracyclines, epidophillotoxins, or anti-metabolites in combination with the immunoglobulin compositions described herein. Yet another aspect may be the use of protracted maintenance therapy using oral methotrexate combined with mercaptopurine and the subject immunoglobulins. Another alternative contemplates the use of prophylactic intrathecal chemotherapy (with or without cranial radiotherapy) for CNS prophylaxis in combination with the subject immunoglobulins. For additional information on the therapy modalities for treating leukemia that can be used in combination with the instant invention, *see* Issa Khouri et al., "Molecular Biology of Leukemias," in CANCER: PRINCIPLES & PRACTICE OF ONCOLOGY 2285-2293 (Vincent T. DeVita et al., editors, 5th ed., 1997); David A. Scheinberg et al., "Acute Leukemias," in CANCER: PRINCIPLES & PRACTICE OF ONCOLOGY 2293-2321; and Albert B. Deisseroth et al., "Chronic Leukemias," in CANCER: PRINCIPLES & PRACTICE OF ONCOLOGY 2321-2343.

### 4.4 Combination Therapy For Treating Metastatic Disease

Treatment of metastases can be with the compositions, combination therapies and methods described herein by themselves or in combination with other cancer treatment modalities depending on the site of the metastases and the primary tumor from which the metastases originates. The most common sites for tumors to metastasize are brain, lung, liver, bone, malignant pleural and pericardial effusions, and malignant ascites.

### 4.4.1 Brain Metastases

Brain metastases develop when tumor cells that originate in tissues outside the central nervous system (CNS) spread secondarily to directly involve the brain. Intracranial metastases may involve the brain parenchyma, the cranial nerves, the blood vessels (including the dural sinuses), the dura, the leptomeninges, and the inner table of the skull. Of the intracranial metastases, the most common are intraparenchymal metastases. The frequency of brain metastasis by primary tumor is lung (48%), breast (15%), melanoma (9%), colon (5%), other known primary (13%); and other unknown primary tumors (11%). *See* Jay S. Loeffler, et al., "Metastatic Brain Cancer," in CANCER: PRINCIPLES & PRACTICE OF ONCOLOGY 2523-2536 (Vincent T. DeVita et al., editors, 5th ed., 1997). Symptoms associated with brain metastasis include altered mental status, hemiparesis, hemisensory loss, papilledema, and gait ataxia. Thus, patients newly diagnosed with brain metastases are often placed on anticonvulsant prophylaxis and corticoseteroids for prolonged periods of time. Such drugs include phenytoin sodium and phenobarbital.

Brain metastases can be treated surgically with excision of the metastases if they are easily reached. With the advancement in imaging and localization techniques, the morbidity associated with surgical removal of brain metastases has decreased. However, risks still remain. Radiotherapy is therefore a mainstay of the treatment of patients with brain metastases. Radiotherapy may be combined with surgery as an adjuvant treatment to surgery. Alternatively, radiosurgery may be used. Radiosurgery is a technique of external irradiation that uses multiple convergent beams to deliver a high single dose of radiation to a small volume. Radiotherapy may be administered in combination with other chemotherapeutic agents such as methyl-CCNU or ACNU or a combination of radiotherapy, methyl-CCNU, ACNU, and tegafur (an orally administered 5'fluorouracil).

Chemotherapy can also be used with brain metastases. Depending on the primary tumor, any of one or combination of the following agents may be administered to the patient: cyclophosphamide, 5-fluorouracil, vincristine, methotrexate, doxorubicin, prednisone, and adriamycin (*e.g*., in a combination of CAF or CMF).

The antibody compositions and methods of using the antibodies as disclosed herein can be combined with any of the conventional therapy modalities described herein or known in the art (*see*, *e.g*., Jay S. Loeffler, et al., 1997) for brain metastases.

### 4.4.2 Lung Metastases

The lungs are the second most frequent site of metastatic disease. Anatomically, the lungs are vascular rich sites and the first capillary bed encountered by circulating tumor cells as they exit from the venous drainage system of their primary tumor. Thus, the lungs act as the initial filtration site, where disseminated tumor cells become mechanically trapped. However, the cells which get trapped there and go on to proliferate and form metastatic lesions will largely depend upon the original primary tumor from which they derive. This hematogenous process of lung metastases is the most common means, but pulmonary metastases can also occur via the lymphatic system. *See* Harvey I. Pass et al., "Metastatic Cancer to the Lung," in CANCER: PRINCIPLES & PRACTICE OF ONCOLOGY 2536-2551 (Vincent T. DeVita et al., editors, 5th ed., 1997).

The most common primary tumors which go on to have lung metastases include soft tissue sarcoma, colorectal carcinoma, germ cell tumors, osteosarcoma, certain pediatric tumors (*e.g*., rhabdomyosarcomas, Ewing's sarcomas, Wilm's tumor, liposarcomas, leiomyosarcomas, alveolar sarcomas, synovial sarcomas, fibrosarcomas, neurogenic sarcomas, and epithelial sarcomas), melanoma, renal cell carcinoma, and breast carcinoma. Most of the metastases from these primary tumors are treated surgically. However, some recommend surgery in combination with chemotherapy. For example, germ cell tumors which have metastasized to the lung are treated with surgical resection following curative cisplatin-based combination chemotherapy.

Treatment of lung metastases frequently involves metastasectomy (*i.e*., surgical removal of the lung metastatic lesion). Thus one aspect of the invention contemplates the use of the disclosed antibodies in combination with conventional therapies, as discussed herein or as known in the art, for the treatment of lung metastases. For additional treatment modalities, *see*, *e.g*., Harvey I. Pass et al., 1997.

Thus, an aspect of the invention contemplates combining anti-α4 integrin immunoglobulins or immunoglobulins that recognize and bind to α4 ligands (*e.g*, VCAM-1 and MadCAM-1) and methods of use with any available treatment modalities for treating lung metastases.

### 4.4.3 Liver Metastases

Metastatic disease in the liver can occur from many primary tumor sites. Because of anatomic venous drainage, gastrointestinal tumors spread preferentially to the liver, such that many patients are initially diagnosed with cancer in the liver. With most gastrointestinal tumors that metastasize to the liver, the diagnosis is dire with relatively short survival. But, colorectal metastases to the liver may be amenable to treatment after resectional therapy.

Systemic chemotherapy represents the modality most frequently used in the treatment of hepatic metastases. Response to systemic chemotherapy varies depending on the primary tumor. Another therapy option is hepatic arterial chemotherapy. Liver metastases are perfused almost exclusively by the hepatic artery, while normal hepatocytes derive their blood from both the portal vein and the hepatic artery. Thus, hepatic arterial chemotherapy, wherein 3H-floxuridine (3H-FUDR) (or other chemotherapeutic agent or agents) is injected into the hepatic artery, results in significantly increased drug concentrations (15 fold) in the metastases than in normal liver tissue. Additional drugs administered via the hepatic artery include but are not limited to fluorouracil, 5-fluorouracil-2-deoxyuridine, bischlorethylnitrosourea, mitomycin C, cisplatin, and doxorubicin.

For a metastasis to the liver, treatment modalities can include systemic chemotherapy (using for example 3H-floxuridine), intrahepatic therapy, hepatic artery ligation or embolization, chemoembolization, radiation therapy, alcohol injection, and cryosurgery. For chemoembolization, the following drug regimens can be used (1) DSM and mitomycin C; (2) collagen, cisplatin, doxorubicin, and mitomycin C; (3) fluorouracil, mitomycin C, ethiodized oil, and gelatin; (4) angiostatin (or other drug which inhibits neovascularization or angiogenesis), cisplatin, doxorubicin, and mitomycin C; (5) lipiodol and doxorubicin; (6) gel foam, doxorubicin, mitomycin C, and cisplatin; (7) doxorubicin, mitomycin C, and lipiodol; and (8) polyvinyl, alcohol, fluorouracil, and interferon. For additional treatments and detail, *see* John M. Daly et al., "Metastatic Cancer to the Liver," in CANCER: PRINCIPLES & PRACTICE OF ONCOLOGY, 2551- 2569 (Vincent T. DeVita et al., editors, 5th ed., 1997).

For metastases to the liver, any of the available treatment modalities can be used in combination with the methods and compositions comprising the immunoglobulins of the subject invention.

### 4.4.4 Bone Metastases

Treatment of bone metastases is best approached using a multimodality methodology. One of the problems with bone is the incidence of bone fracture and bone healing. Tumor mass for bone tumors can be performed surgically and can include amputation of a limb. In addition to surgical treatment, radiation can be used on skeletal metastases. Localized external radiation, hemibody radiation, or systemic radionuclide therapy can be considered for widely disseminated bone disease. Bone seeking isotopes such as 89Sr are advocated as they are better tolerated than 32P-orthophosphate, which is a high energy isotope. For additional modalities and details for treating bone metastases, *see*, *e.g*., John H. Healy, "Metastatic Cancer to the Bone," in CANCER: PRINCIPLES & PRACTICE OF ONCOLOGY 2570-2586 (Vincent T. DeVita et al., editors, 5th ed., 1997).

For metastases to the bone, any of the available treatment modalities can be used in combination with the methods and compositions using anti-α4 integrin immunoglobulins or antibodies against ligands that bind to a α4 integrin (*e.g*., VCAM-1 and MadCAM-1).

### 4.5 Combination Therapy for Ameliorating Conditions Associated With Treating Cancer

Generally, the idea behind the original cancer treatment modalities using radiation or chemotherapy is to poison cancer cells which proliferate faster than normal cells, making them more susceptible to the chemotherapy and radiation. Treating a patient with radiation and chemotherapy or even with some of the newer cancer treatment modalities however, does have adverse side effects to the patient being treated. Thus, one aspect of the invention contemplates the use of compounds and compositions which ameliorate the negative effects produced by the combination of the treatment modalities used to treat the patients. For example, drugs can be administered to the patient in conjunction with the anti-cancer therapy that would treat adverse effects such as but not limited to nausea, vomiting, mucositis and other oral complications, cystitis, pulmonary toxicity, cardiac toxicity, hair loss, and gonadal dysfunction. Although some of these reagents may appear purely cosmetic (*e.g*., inhibition of hair loss), studies have shown that maintaining a positive attitude toward cancer treatment and preventing depression can aid overall treatment response by the patient being treated. Accordingly, the reagents and combination treatments discussed herein can be further combined with drug treatments that ameliorate these adverse effects, as well as in combination with any conventional cancer treatment modalities. For details regarding methods of ameliorating the adverse effects of cancer therapies, *see* generally CANCER: PRINCIPLES & PRACTICE OF ONCOLOGY (Vincent T. DeVita et al., editors, 5th ed., 1997).

### 5. Immunoglobulin Formulations and Methods of Administration

One aspect of the invention contemplates the use of anti-α4 immunoglobulins or immunoglobulins that bind to a α4 ligand (e.g, VCAM-1 and MadCAM-1). Such immunoglobulins may be complete antibodies, antibody fragments, or recombinantly produced immunoglobulins that recognize and bind to these polypeptides.

The antibodies or immunoglobulins of interest discussed above preferably are administered in a physiologically acceptable carrier to a subject. The antibodies may be administered in a variety of ways including but not limited to parenteral administration, including subcutaneous (s.c.), subdural, intravenous (i.v.), intramuscular (i.m.), intrathecal, intraperitoneal (i.p.), intracerebral, intraarterial, or intralesional routes of administration, localized (*e.g*., surgical application or surgical suppository), and pulmonary (*e.g*., aerosols, inhalation, or powder) and as described further below. Preferably, the anti-α4 immunoglobulins or immunoglobulins to the ligands of α4 are administered intravenously or subcutaneously.

Depending upon the manner of introduction, the immunoglobulins may be formulated in various ways. The concentration of therapeutically active immunoglobulin in the formulation (*i.e*., a formulation that is therapeutically effective to the subject to which it was administered) may vary from about 0.01 mg/mL to 1 g/mL. Preferably, the immunoglobulin composition, when administered to a subject in need thereof, reaches a concentration in the blood of the subject to whom it was administered of about 10 µg/mL or more.

Preferably, the immunoglobulin is formulated for parenteral administration in a suitable inert carrier, such as a sterile physiological saline solution. For example, the concentration of immunoglobulin in the carrier solution is typically between about 1-150 mg/mL. The dose administered will be determined by route of administration. Preferred routes of administration include parenteral, subcutaneous, or intravenous administration.

For parenteral administration, the immunoglobulins of the invention can be administered as injectable dosages of a solution or suspension of the substance in a physiologically acceptable diluent with a pharmaceutical carrier, which can be a sterile liquid such as water and oils with or without the addition of a surfactant. Other acceptable diluents include oils of animal, vegetable, or synthetic origin, for example, peanut oil, soybean oil, and mineral oil. In general, glycols such as propylene glycol or polyethylene glycol (PEG) are preferred liquid carriers, particularly for injectable solutions. The antibodies and immunoglobulins of this invention can be administered in the form of a depot injection or implant preparation, which can be formulated in such a manner as to permit a sustained release of the active ingredient(s). A preferred composition comprises a monoclonal antibody at 20 mg/mL, formulated in aqueous buffer consisting of 50 mM L-histidine, 150 mM NaCl, adjusted to pH 6.0 with HCl.

According to one aspect of the invention, an immunoglobulin that recognizes and binds to α4, an α4 dimer or a ligand which binds to α4 (or its dimer) may be administered alone, or in combination with other agents as discussed above to treat and/ameliorate a tumor. These reagents can also be used in the preparation of a medicament for use in treating a patient. Administration of other cancer therapeutic agents can occur prior to, concurrent with, or after administration with the immunoglobulin. Administration of the subject immunoglobulins can occur before, during or after surgical treatment, radiotherapy, hormone therapy, immunotherapy, hyperthermia, or other cancer treatment modality. Administration of the subject immunoglobulins can occur daily, weekly, or monthly as needed. Preferably, the immunoglobulins are administered weekly for one or more weeks.

Therapeutic formulations of the immunoglobulin are prepared for storage by mixing the immunoglobulin having the desired degree of purity with optional physiologically acceptable carriers, excipients, preservatives, or stabilizers (REMINGTON'S PHARMACEUTICAL SCIENCES, 16th ed., A. Osol, Ed., 1980 and more recent editions), in the form of a lyophilized cake or aqueous solution. Acceptable immunoglobulin carriers, excipients or stabilizers are non-toxic, non-therapeutic and/or non-immunogenic to recipients at the dosages and concentrations employed, and include buffers such as phosphate, citrate, and other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine; glutamine, asparagine, arginine or lysine; monosaccharides, disaccharides, and other carbohydrates including glucose, mannose, or dextrins; chelating agents such as ethylenediaminetetraacetate (EDTA); sugar alcohols such as mannitol or sorbitol; salt-forming counterions such as sodium; and/or non-ionic surfactants such as Tween®, Pluronics or polyethylene glycol (PEG). Specific examples of carrier molecules include but are not limited to glycosaminoglycans (*e.g.*, heparin sulfate), hyaluronic acid, keratan-sulfate, chondroitin 4-sulfate, chondroitin 6-sulfate, heparan sulfate, dermatin sulfate, perlecan and pentopolysulfate.

Pharmaceutical compositions comprising immunoglobulins can also include, if desired, pharmaceutically acceptable, non-toxic carriers or diluents, which are vehicles commonly used to formulate pharmaceutical compositions for animal or human administration. The diluent is selected so as not to affect the biological activity of the combination. Examples include but are not limited to distilled water, physiological phosphate-buffered saline, Ringer's solutions, dextrose solution, and Hank's solution.

The agents of the invention can be formulated into preparations for injections by dissolving, suspending or emulsifying them in an aqueous or non-aqueous solvent, such as vegetable or other similar oils, synthetic aliphatic acid glycerides, esters of higher aliphatic acids or propylene glycol. The formulations may also contain conventional additives, such as solubilizers, isotonic agents, suspending agents, emulsifying agents, stabilizers and preservatives.

The immunoglobulins may also be utilized in aerosol formulation to be administered via inhalation or pulmonary delivery. The agents of the present invention can be formulated into pressurized acceptable propellants such as dichlorodifluoromethane, propane, nitrogen and the like.

The immunoglobulin also may be entrapped in microcapsules prepared, for example, by coacervation techniques or by interfacial polymerization (*e.g.*, hydroxymethylcellulose or gelatin-microcapsules and poly-methylmethacylate microcapsules), in colloidal drug delivery systems (*e.g.*, liposomes, albumin microspheres, microemulsions, nanoparticles and nanocapsules), or in macroemulsions. Such techniques are disclosed in REMINGTON'S PHARMACEUTICAL SCIENCES, *supra.*

The immunoglobulin to be used for *in vivo* administration must be sterile. This is readily accomplished by filtration through sterile filtration membranes, prior to or following lyophilization and reconstitution. The immunoglobulin ordinarily will be stored in lyophilized form or in solution.

Therapeutic immunoglobulin compositions generally are placed into a container having a sterile access port, for example, an intravenous solution bag or vial having a stopper pierceable by a hypodermic injection needle or similar sharp instrument.

Suitable examples of sustained-release preparations include semipermeable matrices of solid hydrophobic polymers containing the protein, which matrices are in the form of shaped articles, *e.g.,* films, or microcapsules. Examples of sustained-release matrices include polyesters, hydrogels (*e.g.*, poly(2-hydroxyethylmethacrylate)) as described by Langer et al., J. Biomed Mater. Res. 15: 167-277 (1981) and Langer, Chem. Tech. 12: 98-105 (1982) or poly(vinylalcohol)), polylactides (U.S. Patent No. 3,773,919), copolymers of L-glutamic acid and gamma ethyl-L-glutamate (Sidman et al., Biopolymers 22: 547-556, 1983), non-degradable ethylene-vinyl acetate (Langer et al., *supra*), degradable lactic acid-glycolic acid copolymers such as the LUPRON DEPOT™ (i.e., injectable microspheres composed of lactic acid-glycolic acid copolymer and leuprolide acetate), and poly-D-(-)-3-hydroxybutyric acid (EP 133,988).

While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for immunoglobulin stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, developing specific polymer matrix compositions, and the like.

Sustained-release immunoglobulin compositions also include liposomally entrapped immunoglobulin. Liposomes containing the immunoglobulin are prepared by methods known per se. *See, e.g.,* Epstein et al., Proc. Natl. Acad. Sci. USA 82: 3688-92 (1985); Hwang et al., Proc. Natl. Acad. Sci. USA 77: 4030-4 (1980); U.S. Patent Nos. 4,485,045; 4,544,545; 6,139,869; and 6,027,726. Ordinarily, the liposomes are of the small (about 200 to about 800 Angstroms), unilamellar type in which the lipid content is greater than about 30 mole percent (mol. %) cholesterol; the selected proportion being adjusted for the optimal immunoglobulin therapy.

The immunoglobulins of this invention can be administered in a sustained release form, for example a depot injection, implant preparation, or osmotic pump, which can be formulated in such a manner as to permit a sustained release of the active ingredient. Implants for sustained release formulations are well-known in the art. Implants are formulated as microspheres, slabs, etc. with biodegradable or non-biodegradable polymers. For example, polymers of lactic acid and/or glycolic acid form an erodible polymer that are well-tolerated by the host. The implant is placed in proximity of a solid tumor for example, so that the local concentration of active agent is increased at that site relative to the rest of the body.

A typical daily dosage might range for immunoglobulins from about 1 µg/kg to up to about 200 mg/kg subject weight or more, more preferably from about 0.01 mg/kg to about 150 mg/kg subject weight, more preferably from about 0.1 mg/kg to about 100 mg/kg subject weight, more preferably from about 1 mg/kg to about 75 mg/kg patient weight (and every integer value between these values) depending on the factors mentioned herein. Typically, the clinician will administer immunoglobulin until a dosage is reached that achieves the desired effect. The progress of this therapy can be easily monitored by conventional assays.

A "stable" immunoglobulin, antibody, or antibody fragment formulation is one in which the protein therein essentially retains its physical stability and/or chemical stability and/or biological activity upon storage. Various analytical techniques for measuring protein stability are available in the art and are reviewed in Peptide and Protein Drug Delivery, 247-301 (Vincent Lee Ed., Marcel Dekker, Inc., New York, N.Y., Pubs. 1991) and A. Jones, Adv Drug Delivery Rev. 10: 29-90 (1993), for example. Stability can be measured at a selected temperature for a selected time period. Preferably, the formulation is stable at room temperature (about 30°C) or at 40°C for at least 1 month and/or stable at about 2-8°C for at least 2 years. Furthermore, the formulation may be stable following freezing (to, *e.g., -* 70°C) and thawing of the formulation.

An immunoglobulin "retains its biological activity" in a pharmaceutical formulation, if the biological activity of the immunoglobulin at a given time is within about 10% (within the errors of the assay) of the biological activity exhibited at the time the pharmaceutical formulation was prepared as determined in an antigen-binding assay, for example.

Although the present invention has been described in detail with reference to examples below, it is understood that various modifications can be made without departing from the spirit of the invention, and would be readily known to the skilled artisan.

### EXAMPLES

### Example 1

### Effects of Natalizumab on Tumor Cell Proliferation In Vitro

In order to assess the potential of natalizumab to induce or inhibit proliferation of transformed cells and clonal expansion possibly leading to neoplasia, experiments were performed to determine the ability of natalizumab to stimulate growth of malignant cells expressing the receptor *in vitro*, and studies were conducted in animal tumor models.

The evaluation of positive or negative effects on proliferation of the tumor cell lines was performed using 3H-thymidine incorporation assays. Evaluation of the inhibition or potentiation of human tumor cell growth and/or metastasis was determined using a tumor xenograft nude/SCID mouse model. Thirty-two tumor cell lines were screened. Of the thirty-two tumor cell lines, twelve were colorectal carcinoma, colorectal adenocarcinoma, colon adenocarcinoma, or colon carcinoma cell lines; seven were melanomas or amelanotic melanomas; six were urinary bladder transitional cell carcinomas or urinary bladder carcinomas; three were leukemias; two were lymphomas; and two were prostate carcinomas.

Immunohistochemistry (IHC) and fluorescence activated cell sorting (FACS) were performed to test for α4 expression. Five of the twenty-six cell lines evaluated were positive by IHC. Four of twenty-one cell lines considered negative or equivocal by immunohistochemistry (IHC) were strongly positive (>85%) by FACS analysis. Two cell lines negative or equivocal by IHC were weakly to moderately positive (22.5 and 77.6%) by FACS analysis. Six cell lines were not evaluated by IHC. Only one cell line was moderately positive by FACS analysis (68.4%)

An adhesion assay for VCAM-1 binding was also performed. Seven of the eleven cell lines evaluated were positive. In a preliminary cell proliferation assay, all nine of the nine cell lines evaluated had no cytotoxic or proliferative effect. AS283, HT-29, MOLT-4, and UM-UC-3 cell lines were treated with Tysabri® (natalizumab). Tritiated thymidine uptake was measured. At 1, 2, 3, 4 and 5 days of exposure at eight different concentrations (0.001-100 mg/mL), no evidence of a cytotoxic or proliferative effect was seen.

### Example 2

### Effects of Natalizumab on growth of subcutaneously-implanted

### human MOLT-4 xenografts in SCID mice

The objective of this study was to determine the effect of the treatment with natalizumab on the growth of subcutaneously-implanted human MOLT-4 acute lymphoblastic leukemia xenografts in female SCID mice.

### Materials and Methods

In-life experimental work commenced on Day 0 and was terminated on Day 80. Five-to-six-weeks-old, female SCID mice were purchased from Taconic Farms, Inc. (Germantown, NY) and acclimated in the laboratories one week prior to experimentation. The animals were housed in a pathogen-free barrier facility in micro-isolator cages, with five animals per cage in a 12-hour light/dark cycle. The animals received filtered Birmingham municipal water and sterile rodent food *ad libitum.* Mice were fed sterilizable rodent diet (Harlan-Teklad TD8656). Cages were changed twice weekly. The animals were observed daily and clinical signs were noted.

Thirty-to-forty milligram fragments of MOLT-4 human leukemia were implanted in mice near the right auxillary area using a 12-gauge trocar needle and allowed to grow. The day of tumor fragment implantation was Day 0 of the experiment. Tumors were allowed to reach 75-144 mg in weight (75-144 mm3 in size) before the start of the treatments. A sufficient number of mice were implanted so that tumors in a weight range as narrow as possible were selected for the trial on the day of treatment initiation (Day 14 after tumor implantation). Those animals selected with tumors in the proper size range were randomized into treatment groups. The mean tumor weights on the first day of treatment were ranging from 84 or 96 mg; median tumor weights were 75 or 88 mg on Day 14.

One donor tumor was harvested on the day of tumor fragments implantation and cut in half. One half of the tumor was frozen in O.C.T. compound and the second half of the tumor was fixed in 10% buffered formalin. Both frozen and formalin-fixed tumor halves were shipped to Biogen Idec Inc. for analysis. One fragment from each donor tumor used for implantation of mice was placed in a tube with thioglycollate medium and incubated at +37°C for 24 hours.

Natalizumab (a 20-mg/mL solution, Gensia Sicor Pharmaceuticals Inc., Lot Nos. F23014 and G23004) and a diluent (Placebo for Tysabri®, Gensia Sicor Pharmaceuticals Inc., Lot No. F85002) were received from Biogen Idec Inc. and were stored at +2-8°C upon receipt. Vials with a 0.97-mg/mL solution of human immunoglobulin (IgG4) were supplied by Sigma-Aldrich (St. Louis, MO, Lot No. 122K9159) and were stored at -84°C upon receipt. A clinical formulation of Taxol® (placlitaxel) (6.0 mg/mL in 50% cremophor-EL/50% ethanol, Bristol-Myers Squibb Company, Princeton, NJ; Lot No. 2L57296) was purchased and refrigerated upon receipt. Saline (Saline Solution 0.9%, for animal use only) was purchased from Phoenix Pharmaceutical, Inc. (St. Joseph, MO, Lot Nos. 262053F, 206205F, 208281F) and stored at room temperature. A new bottle of saline was used on each day of formulation.

On Day 14, a 20-mg/mL solution of natalizumab was diluted with Placebo for Tysabri® to 1.0 mg/mL. On subsequent injection days, a 0.5-mg/mL solution of natalizumab was formulated by diluting a 20-mg/mL stock solution with Placebo for Tysabri®. Care was taken not to shake the vials with natalizumab; the vials were inverted to mix. A new vial of natalizumab and Placebo for Tysabri® were used on each day of formulation. On Day 14, a supplied 0.97-mg/mL stock solution of IgG4 was thawed and injected into mice. On subsequent injection days, a 0.5-mg/mL solution of IgG4 was prepared by diluting a 0.97-mg/mL stock solution with Saline Solution 0.9%. A new vial with IgG4 was used on each day of formulation. An aliquot of a commercially formulated 6-mg/mL solution of Taxol® in 50% cremophor-EL/50% ethanol was diluted on each day of injection with Saline Solution 0.9% to make a 1.5-mg/mL solution in 12.5% cremophor-EL/12.5% ethanol/75% saline. All dosing solutions were injected within 2 to 3 hours of formulation.

On Days 14, 17, 24, 31, 38, and 45 two 1-mL aliquots of dosing solution for Group 1 (saline) and Group 2 (natalizumab) were withdrawn from the dosing bottle immediately after the completion of the formulation. Each 1-mL aliquot of natalizumab and saline was dispensed into a vial labeled with the study name, group number, compound name, dosage, route of administration, injection volume, day of the study, and words "Prep. Lab." and vials were refrigerated immediately. Additional two 1-mL aliquots of the same dosing solution of saline and natalizumab were drawn into two syringes per group (1 mL/syringe) before the initiation of the injections of each group, and the syringes were allowed to sit at room temperature during dosing of all twenty animals in the group. After the completion of treatment, the contents of each syringe were dispensed into a vial labeled with the study number, group number, compound name, dosage, route of administration, injection volume, day of the study, and words "Animal Lab." and the vials were refrigerated immediately. After the completion of the experiment these aliquots were shipped to Covance Laboratories, Inc. for analysis.

### Drug Treatment

Animals were randomly divided into six groups of 20 animals each and one group of 30 animals. Two groups (Groups 1 and 5) were treated with Saline Solution 0.9% (saline). Animals in Groups 2, 6, and 7 were treated with natalizumab. Animals in Group 3 were treated with IgG4 (isotype control). Animals in Group 4 were treated with Taxol® (positive control). Treatments with saline, natalizumab, and IgG4 (Groups 1, 2, 3, 5, and 6) were administered IP on Days 14, 17, 21, 24, 28, 31, 35, 38, 42, and 45 (Q3D x 2 for five weeks, Friday - Monday schedule). Treatment with natalizumab in Group 7 (a group of 30 mice) was administered on Days 14, 17, 21, 24, 28, 31, 35, and 38 (Q3D x 2 for four weeks). On Day 14 natalizumab was administered at a dosage of 10 mg/kg, followed by further treatments with a dosage of 5 mg/kg/dose. On Day 14 IgG4 was administered at a dosage of 9.7 mg/kg, followed by further treatments with a dosage of 5 mg/kg/dose. Taxol® was administered intravenously (IV) once a day for five consecutive days (Q1D x 5) at a dosage of 15 mg/kg/dose starting on Day 14. All test compounds were administered by exact individual animal body weight on each day of treatment in a vehicle volume of 0.1 mL/10 g body weight.

The animals were weighed and the subcutaneous (SC) tumors were measured on the days when treatments were administered starting on Day 14 with an exception of two measurements which were done one day before the injection (on Days 24 and 41). After the completion of treatment the tumor and body weight data were collected twice a week. Tumor volume was determined by caliper measurements (mm) and using the formula for an ellipsoid sphere: LxW2/2 = mm3, where L and W refer to the larger and smaller perpendicular dimensions collected at each measurement. This formula was also used to calculate tumor weight, assuming unit density (1 1 mm3 = 1 mg).

Blood of animals in Group 7 was collected over the course of the treatment. Animals No. 1-5 were bled 8 hours after the injection on Day 14. Five animals were bled prior to injection on Day 21 (Animals No. 6-10), Day 28 (Animals No. 11-15), Day 35 (Animals No. 16-20), and Day 38 (Animals No. 21-25). Animals No. 26-30 were bled 8 hours after the injection on Day 38. Blood was collected by retro-orbital puncture under CO₂/O₂ anesthesia. Animals were bled for the maximum amount of blood (terminal bleeding) using an uncoated capillary tube and blood was allowed to clot at room temperature for 30 minutes from the time the last animal at each time point was bled. Blood samples then were centrifuged at 10,000 rpm for 10 minutes at +4°C; serum was separated and frozen on dry ice. All serum samples were stored at -84°C until shipped to Biogen Idec for analysis.

Primary tumors of animals in Groups 5 and 6 were surgically excised on Day 28, one day after the median tumor weight in Group 5 reached 466 mg. Administration of anesthesia and surgery/post-surgery recovery procedures were conducted following approved standard laboratory methods. Briefly, each animal was anesthetized and the incision site was prepared with an antiseptic solution. An incision along the periphery of one side of the tumor (no more than 3-4 mm longer than the diameter of the tumor) was made, the skin was reflected and the tumor was everted through the incision. Tumor was dissected away from the skin and muscle. The incision was closed with a 4.0-mm wound clip. Each excised tumor was cut in half, with one half of the tumor frozen in O.C.T. compound and the second half of the tumor preserved in 10% buffered formalin. The site of the resection was monitored for tumor re-growth twice a week and when a new solid tumor was formed, its dimensions were recorded.

Animals No. 1-10 in Groups 1-4 were euthanized on Day 47 after tumor implantation (16 May 2003); Animals No. 11-20 in Groups 1-4 were euthanized on Day 48 (17 May 2003). All surviving animals in Groups 5 and 6 (except for Animal No. 12 in Group 5 and Animal No. 8 in Group 6, for explanations see below) were euthanized on Day 80 after tumor implantation (19 May 2003). Animal No. 12 in Group 5 was sacrificed on Day 73 due to excessive tumor weight (>4,000 mg). Animal No. 8 in Group 6 was sacrificed on Day 73 due to tumor ulceration. Five animals in Group 7 were euthanized on Days 14, 21, 28, 35, and ten animals were euthanized on Day 38. Carcasses of all animals alive on Days 47, 48, and 80 were submitted to the Safety Assessment Department of Southern Research Institute for necropsy followed by complete histopathological evaluation. Animals in Group 7 were excluded from necropsy and complete histopathological evaluation. For the animals in Groups 1-6, tumor implantation (subcutaneous) took place on Day 0. Treatment was initiated on Day 14, and treatment was terminated on Day 45. Primary tumor excision occurred on Day 28 post-implantation in Groups 5 and 6. The animals were sacrificed on Day 80. Animals in the non-excision group were sacrificed at days 47-48.

**Table 1**

| **Group** | **Cell Line** | **Treatment** | **N** | **Dose (mg/Kg)** | **Dose Vol.(mL/kg)** |
|---|---|---|---|---|---|
| 1 | MOLT-4 | Saline | 20 | 0 | 10 |
| 2 | MOLT-4 | Taxol | 20 | 15 | 10 |
| 3 | MOLT-4 | hIgG4 | 20 | 10/5 | 10 |
| 4 | MOLT-4 | Natalizumab | 20 | 10/5 | 10 |

| **Groups** | **Cell Line** | **Treatment** | **N** | **Dose (mg/k)** | **Dose Vol.(mL/kg)** |
|---|---|---|---|---|---|
| 5 | MOLT-4 | Saline | 20 | 10 | 10 |
| 6 | MOLT-4 | Natalizumab | 20 | 10/5 | 10 |

For the animals in Groups 7-10, tumor implantation (subcutaneous) took place on Day 0. Treatment was initiated on Day -7, and treatment was terminated on Day 45. The animals were sacrificed on Days 46-47.

**Table 2**

| **Group** | **Cell Line** | **Treatment** | **N** | **Dose (mg/kg)** | **Dose Vol.(mL/kg)** |
|---|---|---|---|---|---|
| 7 | MOLT-4 | Saline | 20 | 0 | 10 |
| 8 | MOLT-4 | Taxol | 20 | 15 | 10 |
| 9 | MOLT-4 | hIgG4 | 20 | 10/5 | 10 |
| 10 | MOLT-4 | Natalizumab | 20 | 10/5 | 10 |

For the animals in Group 11, tumor implantation (subcutaneous) took place on Day 0. Treatment was initiated on Day 14, and treatment was terminated on Day 45. Five animals per group sacrificed on days 1, 7, 14, 21, 24, 24. This group was used for verification of natalizumab serum levels.

**Table 3**

| **Group** | **Cell Line** | **Treatment** | **N** | **Dose (mg/kg)** | **Dose Vol.(mL/kg)** |
|---|---|---|---|---|---|
| 11 | MOLT-4 | Natalizumab | 30 | 10/5 | 10 |

Natalizumab administration was initiated on a specified study day with a loading dose of 10 mg/kg. Subsequent doses were administered twice per week (*i.e.,* every 3 - 4 days). Dose levels were selected based on modeling of the pharmacokinetics after a single dose, and the trough serum concentrations in a confirmatory repeated dose pharmacokinetic study. The dose level was selected to provide a minimum serum concentration of 20 ml/mL upon repeated dose administration.

Natalizumab treatment following establishment of the MOLT-4 tumor resulted in slowed tumor growth in the excision group, and some animals in the excision group were tumor free following treatment. Natalizumab treatment initiated prior to MOLT-4 tumor implantation resulted in a significant decrease in tumor growth. Natalizumab treatment did not adversely affect animal health as evidenced by the increased body weights over the course of the treatment period.

The number of non-specific deaths, number of partial and complete tumor regressions, number of tumor-free survivors, and the individual animal's time to reach the evaluation size (time to reach four tumor mass doublings) were determined. The individual animal's time to reach four tumor mass doublings was used in the calculations of the overall delay in the growth of the median tumor (T-C). Group 1 (saline-treated animals) was used a control group (C) in all calculations. Median tumor weights, mean tumor weights and standard deviations, and comparison of the median and mean tumor weight in the treatment groups (T) to the median and mean tumor weight in the control group (C) (MEDIAN T/C = median T/median C x 100% and MEAN T/C = mean T/mean C x 100%, respectively) were calculated for each group on each day of data collection (values being presented in Tables 8-2 through 8-8). Mean body weights and standard deviations were calculated for each group on each day of data collection (values are presented in Tables 8-9 through 8-15).

Tumors of Animals No. 1-10 in Groups 1, 2, 3, and 4 were harvested on Day 47. Tumors of Animals No. 11-20 in Groups 1, 2, 3, and 4 were harvested on Day 48. The primary tumor of Animals No. 1-20 in Groups 5 and 6 was surgically excised on Day 28. The secondary tumor (which re-grew at the site of the primary tumor) of all surviving animals in Groups 5 and 6 was harvested on Day 80. Animals No. 1 and 15 in Group 6 did not have a secondary tumor at necropsy on day 80. (Animal No. 4 in Group 5 and Animal No. 3 in Group 6 died during post-surgery recovery on Day 28, and; thus, did not have a secondary tumor. Animal No. 19 in Group 5, which was found dead on Day 47, was necropsied post-mortem. Animal No. 12 in Group 5 was removed from the experiment on Day 73 due to excessive tumor weight (4224 mg). Animal No. 8 in Group 6 was removed from the experiment on Day 73 due to tumor ulceration. Animals No. 10, 17, and 20 in Group 5 were found dead on Day 73 and were not necropsied due to significant autolysis.)

Each tumor weighing more than 100 mg was cut in half and one half of the each tumor was frozen in O.C.T. compound using a dry ice/ethanol slush, and stored at -84°C. Tumors weighing less than 100 mg (tumors of Animals No. 1, 3, 7, 9, 11, 12, 14-18, and 20 in Group 4) were frozen in O.C.T. compound *in toto.* Tumor of Animals No. 2, 4, 5, 6, 8, 13, and 19 in Group 4, which weighed less than 100 mg, were preserved in 10% buffered formalin in toto. After the completion of the experiment all tumor samples frozen in O.C.T. compound (a total of 145 tumor samples) were shipped to Sierra Biomedical, Division of Charles River Laboratories, to be examined by immunohistochemistry to verify that MOLT-4 was expressing VLA-4 *in vivo* and thus was a valid tumor model for this study. Immunohistochemistry was performed with commercial anti-CD49d antibody and froze sections of MOLT-4 tumors from a subset of animals in each treatment group. The second half of each tumor, if present, was fixed in 10% buffered formalin and submitted to the Safety Assessment Department of Southern Research Institute for histopathological evaluation.

### Data Analysis

Individual tumor measurements and body weights were collected and processed using software ADAS (Automated Data Acquisition System) developed at Southern Research Institute. The data then were exported into MS Excel for reporting purposes. SigmaPlot™ was used to graphically present the raw data. The individual animal's tumor weight on the day of the last measurement prior to termination (Day 47 for Groups 1-4 and Day 80 for Groups 5 and 6) was used as the endpoint in order to statistically compare the growth data between groups. SigmaStat™ software was used to perform the Student's t-test. The Mann-Whitney rank sum test was used in place of a t-test when the data set did not pass the normality or equal variance test. A copy of the results of the statistical analysis is attached to this report.

### Dose Solution Analysis

Two dose solutions (MP-21520 and MP-21522) marginally exceeded the target concentration ± 15% with a +16% and a +19% differential, respectively. These differences were attributed to the 280 nm absorbance of 0.01-0.04 units present in the saline dosing solutions and diluent. This absorbance was attributed to saline extractables in the sample container stopper.

### Growth of SC MOLT-4 Leukemia Xenografts

No growth was observed in any of the tubes with thioglycollate medium in which tumor fragments were placed on the day of tumor fragment implantation and incubated at +37°C for 24 hours. There was a 95% tumor xenograft take rate in the control, saline-treated group (Group 1) with one xenograft out of twenty failing to grow (no-take). Animal No. 20 in Group 1 with a tumor no-take was excluded from all calculations. Tumor growth and administration of saline was tolerated without body weight loss. The median tumor weight reached 1764 mg on Day 47 and achieved four tumor mass doublings in 30.2 days.

Tumors of all twenty saline-treated animals in Group 5 grew progressively after implantation. Tumors were surgically excised on Day 28. One animal died during the post-surgery recovery period (Animal No. 4). Animals lost weight immediately following the surgery (a maximum average body weight loss of 2 g [10%]) but later re-gained weight and continued to grow. The observed body weight loss could be attributed to the administration of anesthesia as well as the net loss of the weight of the excised tumor. Four animals died during the course of the experiment: Animal No. 19 was found died on Day 47; Animals No. 10, 17, and 20 were found died on Day 73. Tumors of all animals re-grew at the site of the primary tumor following the excision. The median tumor weight reached 2363 mg on Day 80 and the median tumor reached four tumor mass doublings in 60.9 days.

### Effect of natalizumab

Administration of natalizumab (Group 2) was well tolerated without deaths and was associated with an average body weight fluctuation of 1 g (5%) during the first week of treatment. All twenty tumors grew over the course of the experiment. The median tumor weight reached 1240 mg on Day 47 and the median tumor reached four tumor mass doublings in 33.5 days. Comparison of the individual tumor weights in the control, saline-treated group (Group 1) and natalizumab-treated group (Group 2) on Day 47 revealed that tumor weights in the natalizumab-treated group were not statistically different when compared with the weights of the tumors in the saline-treated group (p=0.1032, Mann-Whitney rank sum test), indicating that tumors in both groups grew at a similar rate.

Tumors of all twenty natalizumab-treated animals in Group 6 grew progressively after implantation. Tumors were surgically excised on Day 28. One animal died during the post-surgery recovery period (Animal No. 3). Animals experienced minimal body weight loss immediately following the tumor excision surgery (a maximum average body weight loss of 1 g [5%]) but subsequently continued to gain weight. Tumors of seventeen out of nineteen animals re-grew at the site of the primary tumor following the excision with two animals staying tumor-free on the day of study termination, Day 80. The median tumor weight reached 1006 mg on Day 80, with the median tumor not reaching four tumor mass doublings (>66.0 days). Comparison of the individual animal's weights in the saline-treated group (Group 5) and natalizumab-treated group (Group 6) revealed that tumor growth in the natalizumab-treated group was statistically different from the growth of the tumors in the saline-treated group (P=0.0189, t-test), indicating that natalizumab-treated tumors re-grew at a slower rate than tumors in the saline-treated group. Tumors of all thirty natalizumab-treated animals grew in Group 7. Growth of the tumors was not evaluated as animals were periodically removed from the study for terminal blood collection.

### Effect of IgG4

Administration of IgG4 (Group 3) was well tolerated without deaths or body weight loss. All twenty animals had tumor at the termination of the experiment. The median tumor weight reached 1731 mg on Day 47 and the median tumor reached four tumor mass doublings in 31.7 days. Comparison of the individual tumor weights in the control, saline-treated and IgG4-treated groups on Day 47 revealed that tumor weights in the IgG4-treated group were not statistically different when compared with the weights of the tumors in the control group (p=0.8004, Mann-Whitney rank sum test), indicating that tumors in both groups grew at a similar rate.

### Effect of Taxol®

Administration of Taxol® (Group 4) was well tolerated without deaths or sustained body weight loss. An average maximum body weight loss of 5% (1 g) was observed after the end of the treatment (on Days 17 and 21), which is expected because Taxol®, a cytotoxic agent, was administered at an approximate maximum tolerated dosage. Animals regained weight during the post-treatment period. The Taxol® treatment was very effective in the inhibition of the MOLT-4 tumor xenograft growth. Twelve out of twenty animals had no measurable tumor at the termination of the experiment and only three out of eight measurable tumors doubled their mass once over the course of the experiment. Growth of the Taxol®-treated tumors was statistically different from the growth of the tumors in the vehicle-treated, control group (p<0.0001, Mann-Whitney rank sum test), indicating that Taxol® treatment inhibited tumor growth.

### Summary of the Results of the Histopathologic Evaluation

In summary, gross lesions involving the uterus, ovaries, lymph nodes, lung, liver and enlargement of the spleen were observed randomly among the different treatment groups, but none of the findings were considered to be treatment related. Microscopic lesions that occurred randomly in mice from the various treatment groups included extramedullary hematopoiesis in the spleen and liver, focal necrosis and inflammation in the liver, inflammation in the mesentery, and mineralization of the epicardium of the heart. All of these changes were considered to be incidental or spontaneous and unrelated to administration of all test articles. Enhancement of xenograft growth was not evident in any treatment group. Extension of xenograft growth was noted in the ventral abdominal (inguinal) skin of four mice each from Groups 5 and 6. Metastasis or extension of the xenograft was observed in the regional lymph nodes (axillary, inguinal, iliac, mandibular, and mediastinal) of mice in Group 5 and the inguinal lymph node of mice in Group 6. Thymic metastasis was seen in one mouse each from Groups 5 and 6. There was no evidence of exacerbation of growth or metastasis of the secondary human acute lymphoblastic leukemia as a result of the treatment with natalizumab.

### Summary of the Immunohistological Analysis of Tumors

Tumor sections were examined by immunohistochemistry to verify that MOLT-4 was expressing VLA-4 *in vivo* and thus was a valid tumor model for this study. Immunohistochemistry was performed with commercial anti-CD49d antibody and frozen sections of MOLT-4 tumors from a subset of animals in each treatment group. CD49d-specific immunopositivity of MOLT-4 cells was characterized by robust, granular to wispy, diffuse cytoplasmic and cell surface staining. Diffuse cytoplasmic staining suggests that intracytoplasmic, as well as cell surface CD49d, was binding the anti-CD49d antibody. Tumor sections from animals in the saline control group and the IgG4 control group had robust staining and were given immunohistochemical staining scores of 3 to 4 ("Clustered cells, closely spaced" to "Densely packed cells", respectively). Tumor sections from animals in the natalizumab treated group with a prolonged non-treatment period prior to necropsy (Group 6) also had robust staining and were given immunohistochemical staining scores of 3 to 4. In animals treated with natalizumab and sacrificed upon termination of treatment, immunohistochemical staining for CD49d was greatly reduced to absent and tumor sections were given immunohistochemistry scores of 0 to 2 ("No labeled cells" to "Scattered individual cells, closely spaced", respectively). Mice treated with the tumorilytic agent Taxol often did not have sufficient remaining tumor mass following treatment to allow evaluation of immunohistochemical staining. These data confirm that the MOLT-4 human leukemia cell line did express CD49d *in vivo* under the experimental conditions of this study.

### Conclusions

Growth of SC-implanted MOLT-4 human acute lymphoblastic leukemia xenografts was not inhibited by the administration of natalizumab. However, re-growth of the solid tumor at the site of the excision of the primary tumor was inhibited by the administration of natalizumab. Administration of IgG4 had no effect on the growth of the xenografts. The treatment with Taxol® inhibited primary tumor growth, resulting in twelve out of twenty animals having no measurable tumor at the termination of the experiment. Histopathological evaluation of the animals revealed that there was no evidence of exacerbation of growth or metastasis of the human acute lymphoblastic leukemia xenografts as a result of treatment with natalizumab or IgG4. A variety of gross and microscopic lesions occurred with random distribution patterns in all treatment groups. However, all of the findings of lesions were considered spontaneous or incidental and unrelated to saline, natalizumab, IgG4 or Taxol® treatment.

All references cited above are incorporated herein in their entirety for all purposes. This application is related to U.S. Provisional Application No. 60/541,946, filed February 6, 2004, which is herein incorporated in its entirety for all purposes.

## Claims

1. An anti-α4 immunoglobulin for use in a method of inhibiting tumor growth and/or metastatic progression and/or development of metastases in a subject.

2. The immunoglobulin for the use of claim 1, wherein the anti-α4 antibody binds to α4β1 integrin and/or α4β7 integrin.

3. The immunoglobulin for the use of claim 1, wherein the anti-α4 immunoglobulin is natalizumab.

4. The immunoglobulin for the use of claim 1, wherein the tumor is a melanoma, a prostate cancer, a leukemia, or a lymphoma; preferably wherein the melanoma is a cutaneos melanoma, a metastatic melanoma, or an intraocular melanoma; preferably wherein the lymphoma is a non-Hodgkin's lymphoma, a cutaneous T-cell lymphoma, or Hodgkin's disease; preferably wherein the leukemia is chronic myelogenous leukemia, acute myelogenous leukemia, adult acute lymphoblastic leukemia, mature B-cell acute lymphoblastic leukemia, chronic lymphocytic leukemia, prolymphocytic leukemia, or hairy cell leukemia.

5. The immunoglobulin for the use of claim 3, wherein the natalizumab is administered in an amount of about 1 mg/kg subject weight to about 100mg/kg subject weight, preferably wherein the natalizumab is administered in an amount of about 1 mg/kg subject weight to about 20mg/kg subject weight, more preferably wherein the natalizumab is administered in an amount of about 1 mg/kg subject weight to about 10mg/kg subject weight.

6. The immunoglobulin for the use of claim 4, wherein the tumor is a melanoma and the natalizumab is administered after surgical excision of the melanoma.

7. The immunoglobulin for the use of claim 4, wherein the tumor is a melanoma and the subject is further subjected to surgery, isolated limb perfusion, regional chemotherapy infusion, systemic chemotherapy, or immunotherapy with a second antibody to treat the melanoma.

8. The immunoglobulin for the use of claim 7, wherein the second antibody is an anti-GM2 ganglioside antibody, anti-GD2 ganglioside antibody, or anti-GD3 ganglioside antibody.

9. The immunoglobulin for the use of claim 7, wherein the regional chemotherapy infusion or systemic chemotherapy comprises at least one chemotherapeutic agent selected from the group consisting of: dacarbazine, carmustine, lomustine, tauromustine, fotemustine, semustine, cisplatin, carboplatin, vincristine, vinblastine, vindesine, taxol dibromodulcitol, detorubicin, piritrexim, and interferon.

10. The immunoglobulin for the use of claim 9, wherein the interferon is interferon-α2.

11. The immunoglobulin for the use of claim 1, wherein the metastases is a metastasis to brain, lung, liver, or bone.

12. The immunoglobulin for the use of claim 11, wherein the metastasis is to lung, and the tumor is a melanoma.

13. The immunoglobulin for the use of claim 1, wherein the tumor is a lymphoma and the subject is further treated with one or more chemotherapeutic agents and/or radiotherapy.

14. An anti-α4 integrin immunoglobulin or an immunoglobulin against an α4 integrin ligand for use in a combination therapy for inhibiting tumor growth and/or metastatic progression and/or development of metastases wherein said therapy comprises administering the anti-α4 integrin immunoglobulin or an immunoglobulin against an α4 integrin ligand, and a chemotherapeutic, an immunotherapeutic, and/or radiation therapy.

15. The immunoglobulin for the use of claim14, wherein the anti-α4 immunoglobulin is an anti-α4β1 integrin antibody or an anti-α4β7 integrin antibody.

16. The immunoglobulin for the use of claim 15, wherein the anti-α4 immunoglobulin is natalizumab.

17. The immunoglobulin for the use of claim 15, wherein the anti-α4 immunoglobulin is administered intravenously, intrathecally, or subcutaneously.

18. The immunoglobulin for the use of claim 15, wherein the anti-α4 immunoglobulin is natalizumab and is administered in an amount of about 1 mg/kg subject weight to about 100mg/kg subject weight, preferably wherein natalizumab is administered in an amount of about 10mg/kg subject weight to about 30mg/kg subject weight, more preferably

19. The immunoglobulin for the use of claim 15, wherein the anti-α4 immunoglobulin is administered daily, weekly, or monthly, preferably it is administered weekly.

20. The immunoglobulin for the use of claim 1, wherein the subject is further treated with a chemotherapy, an immunotherapy, surgery, radiation therapy, hyperthermia, or a drug to ameliorate the adverse effects of a cancer therapy.

21. The immunoglobulin for the use of claim 20, wherein the tumor is a melanoma, a leukemia, a prostate cancer, or a lymphoma.
